# EUROPEAN PATENT APPLICATION

(11) **EP 2 340 813 A1**
(43) Date of publication of application: **06.07.2011**
(21) Application number: 11152433.6
(22) Date of filing: 01.12.2004
(51) Int. Cl.: A61K 9/20, A61K 31/55, A61P 25/28

(54) **Solid dosage formulations of galantamine**

(30) Priority: 31.12.2003 US 533571 P; 01.12.2004 US 1609
(62) Divisional of application: 04813097.5
(71) Applicant: Actavis Group PTC ehf., 220 Hafnarfirdi (IS)
(72) Inventor: The designation of the inventor has not yet been filed
(74) Representative: Cole, William Gwyn

(57) **Abstract**

Galantamine formulations substantially free of starch or cellulosic material are described.

## Description

### BACKGROUND

Galantamine ((4a*S*, 6*R*, 8a*S*)-4a,5,9,10,11,12-hexahydro-3-methoxy-11-methyl6H-benzofuro[3a,3,2-ef][2]benzazepin-6-ol) is a known reversible, competitive acetylcholinesterase inhibitor. The compound has been isolated from the bulbs of the Caucasian snowdrops Galantanus woronowi in addition to the common snowdrop Galanthus Nivalis.

Galantamine and its salts have been employed as a pharmaceutically active agent in the treatment of a variety of disorders, including mania, alcoholism, nicotine dependence, and Alzheimer's disease. In particular, galantamine hydrobromide has been used for the treatment of Alzheimer's disease and is currently formulated as film-coated tablets of 4 milligram (mg), 8 mg, and 12 mg doses for twice a day oral administration under the trade name REMINYL.

Previously described formulations of this active agent have certain properties that are not ideal in all situations. Manufacturing problems associated with fast dissolving galantamine hydrobromide tablets have been disclosed in the art. It was determined that using either lactose anhydrous or lactose monohydrate as diluent, and either powdered cellulose or microcrystalline cellulose as disintegrant resulted in segregation of the tablet excipients, resulting in tablets having a variable composition. Using a spray-dried mixture of lactose monohydrate and microcrystalline cellulose (75:25) as the diluent in combination with a disintegrant having a large coefficient of expansion was described as providing a formulation that avoided the segregation problems while at the same time resulting in a targeted dissolution profile. Although one formulation has been developed for fast dissolving galantamine hydrobromide tablet, there exists a continuing need for additional formulations, which can be easily processed, provide product consistency, and compliance with product specifications.

The present invention addresses these and other needs for improved galantamine dosage forms.

### SUMMARY OF THE INVENTION

In one embodiment, a pharmaceutical solid dosage formulation comprises galantamine or a pharmaceutically acceptable salt thereof; and a pharmaceutically acceptable excipient, with the proviso that the formulation does not contain microcrystalline cellulose; and wherein the formulation exhibits a dissolution profile such that after 10 minutes at least about 90% of the galantamine or galantamine salt is released after combining the dosage formulation with 500 ml of purified water at 37°C in Apparatus 2 (USP, < 711 > Dissolution, paddle, 50 rpm).

In another embodiment, a pharmaceutical solid dosage formulations comprises galantamine or a pharmaceutically acceptable salt thereof; and a pharmaceutically acceptable excipient, with the proviso that the solid dosage formulation, excluding an optional coating disposed on the solid dosage formulation, i) does not contain a cellulosic material, or ii) does not contain starch.

In yet another embodiment, an immediate release solid pharmaceutical dosage formulation comprises galantamine or a pharmaceutically acceptable salt thereof; and a pharmaceutically acceptable excipient, with the proviso that the formulation does not contain a lactose-based material and wherein the formulation is directly compressible.

In another embodiment, a process of preparing a pharmaceutical dosage formulation comprises blending galantamine or a pharmaceutically acceptable salt thereof with an excipient and disintegrant to form a preblend; blending the preblend with a glidant and optional additives to form a blend; and forming the blend into tablets using direct compression.

In one embodiment, a pharmaceutical dosage formulation comprises (a) galantamine hydrobromide, (b) compressible excipient or inert excipient, and (c) crospovidone, partially pregelatinized maize starch or a combination of the foregoing.

### DETAILED DESCRIPTION

Disclosed herein are galantamine formulations, specifically galantamine solid oral dosage formulations, methods of preparing such formulations, and methods of treating a patient with Alzheimer's disease with the formulations. It has been determined that directly compressible galantamine formulations prepared in the absence of microcrystalline cellulose exhibit excellent compressibility and dissolution profiles.

The use of the terms "a" and "an" and "the" and similar referents in the context of description (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in a suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

By "oral dosage formulation" is meant to include a unit dosage form prescribed or intended for oral administration.

By "immediate-release", it is meant a conventional or non-modified release form in which greater than or equal to about 75% of the active agent is released within two hours of administration, preferably within one hour of administration.

Disclosed herein are solid dosage formulations comprising galantamine or a pharmaceutically acceptable salt thereof; and a pharmaceutically acceptable excipient, with the proviso that the formulation does not contain microcrystalline cellulose.

In yet another embodiment, a solid dosage formulation comprises galantamine or a pharmaceutically acceptable salt thereof; and a pharmaceutically acceptable excipient, with the proviso that the formulation does not contain cellulosic material.

In yet another embodiment, a solid dosage formulation comprises galantamine or a pharmaceutically acceptable salt thereof; and a pharmaceutically acceptable excipient, with the proviso that the formulation does not contain lactose-based material.

The formulations can further comprise a disintegrant, a glidant, or combinations of the foregoing, as well as optional additives. By proper choice of excipient and disintegrant, dosage formulations can be prepared that are directly compressible into tablets possessing good hardness without problems of friability or capping. The tablets can optionally be coated with a film coating.

The dosage formulations can exhibit a dissolution profile such that after 10 minutes at least about 90% of the galantamine or galantamine salt is released after combining the dosage formulation with 500 ml of purified water at 37°C in Apparatus 2 (USP, < 711 > Dissolution, paddle, 50 rpm).

"Dissolution profile" as used herein, means a plot of the cumulative amount of active agent released as a function of time. The dissolution profile can be measured utilizing the Drug Release Test <724>, which incorporates standard test USP 26 (Test <711> Dissolution). A profile is characterized by the test conditions selected. Thus the dissolution profile can be generated at a preselected apparatus type, shaft speed, temperature, volume, and pH of the dissolution media.

It may be preferred to have a formulation that exhibits a dissolution profile such that after 10 minutes at least about 87%,at least about 90%, at least about 91%, at least about 92%, at least about 93%, at least about 94%, at least about 95%, at least about 96%, at least about 97%, at least about 98%, at least about 99%, or at least about 99.5%, of the galantamine or galantamine salt is released after combining the dosage formulation with 500 ml of purified water at 37°C in Apparatus 2 (USP, < 711> Dissolution, paddle, 50 rpm).

In one embodiment, the dosage formulation exhibits a dissolution profile that is substantially identical to that of REMINYL. By "REMINYL" is meant galantamine hydrobromide formulations manufactured by JOLLC, Gurabo, Puerto Rico or Janssen-Cilag SpA Latina, Italy (tablets); or Janssen Pharmaceutica N.V. Beerse, Belgium (oral solution). Specifically, by REMINYL is meant film coated tablets of galantamine hydrobromide, base equivalent of 4, 8, and 12 mg in the presence of inactive ingredients of colloidal silicon dioxide, crospovidone, hydroxy propyl methylcellulose, lactose monohydrate, magnesium stearate, microcrystalline cellulose, propylene glycol, talc, and titanium dioxide, optionally yellow ferric oxide (4 mg tablet), red ferric oxide (8 mg tablet), or red ferric oxide and FD&C yellow #6 aluminum lake (12 mg tablet).

The dosage formulations contain galantamine or a pharmaceutically acceptable salt thereof "Pharmaceutically acceptable salt" includes derivatives of the disclosed compounds, wherein the parent compound is modified to pharmaceutically acceptable solvates, including hydrates, of such compounds and such salts. Examples of pharmaceutically acceptable salts include, but are not limited to, mineral or organic acid salts including those derived from inorganic acids such as hydrochloric, hydrobromic, sulfuric, sulfamic, phosphoric, nitric and the like, specifically hydrobromic.

When prepared into unit dosage forms, such as a tablet, a pharmaceutically effective amount of galantamine or its corresponding salt can be used. Specifically amounts of galantamine or its corresponding salt per unit dosage form may be about 1 to about 40 mg of galantamine as measured by the amount of free base, specifically about 2 to about 20 mg of galantamine; and yet more specifically about 4 to about 16 mg of galantamine.

Excipients for the formulations are inert substances that may be used as a vehicle for the active agent, optionally in conjunction with other excipients, as long as the resulting formulation meets the dissolution profile desired and/or is directly compressible. Suitable excipients include one or more excipients and excipient combinations. For the formulations free of microcrystalline cellulose, suitable excipients include, for example, lactose-based materials such as spray dried lactose; cellulosic materials excluding microcrystalline cellulose; starches including partially pregelatinized starch, pregelatinized starch, partially hydrolyzed starch, maize starch, potato starch, rice starch, wheat starch, and tapioca starch; sugar such as sucrose, dextrose, fructose, and the like; sugar alcohols such as mannitol, sorbitol, xylitol, and the like; lactitol; saccharides such as dextrates and the like; polysaccharides such as maltodextrin and the like; dibasic calcium phosphate, calcium sulphate, and combinations thereof.

In one embodiment, the solid dosage formulation is free of cellulosic material. As used herein, "cellulosic material" includes any material containing cellulose including microcrystalline cellulose, powdered cellulose, modified cellulose such as ethyl cellulose, cellulose acetate, and the like. Furthermore, as used herein, a "solid dosage formulation" is exclusive of an optional coating material disposed on the solid dosage formulation. Such a coating material may contain any type of cellulosic materials and still be encompassed by the described embodiments.

Also included herein are lactose free formulations. These formulations are free of lactose-based material. As used herein a "lactose-based material" includes any material containing lactose, including lactose monohydrate, anhydrous lactose, lactose impalpable, and the like.

A combination of two or more excipients can be used in the dosage formulations. When used, the total amount of excipients can be up to about 99 weight percent of the total weight of the formulation; specifically about 30 to about 98 weight percent; more specifically about 60 to about 97 weight percent; and yet more specifically about 80 to about 95 weight percent.

A "disintegrant" is meant an agent used in a formulation to aid in the break down of a compacted mass in the presence of a fluid environment. Compounds that behave as disintegrants generally possess the ability to swell or expand upon exposure to the fluid environment. Preferably the disintegrant swells upon exposure to an aqueous environment. Certain traditional tablet excipients may function as a disintegrant (e.g. starch), while other materials provide superior results as a disintegrant, for example, croscarmellose sodium, crospovidone, low-substituted hydroxypropyl cellulose, sodium starch glycolate, alginates, and the like.

The disintegrant can be present in the formulations in an amount of about 0.1 to about 20 weight percent of the total weight of the formulation; specifically about 0.5 to about 10 weight percent; and yet more specifically about 1 to about 8 weight percent.

A lubricant and/or glidant can also be used in the dosage formulations to aid in the processing of powder materials. Exemplary lubricants include calcium stearate, glycerol behenate, magnesium stearate, mineral oil, polyethylene glycol, sodium stearyl fumarate, stearic acid, talc, vegetable oil, zinc stearate, and combinations thereof. Glidants include, for example, silicon dioxide. Certain materials can act as both a glidant and a lubricant.

The lubricant or glidant can be used in amounts of about 0.1 to about 15 weight percent of the total weight of the formulation; specifically about 0.5 to about 5 weight percent; and yet more specifically about 0.75 to about 3 weight percent.

Other optional additives to the dosage formulations include, for example, flavoring agents, stabilizing agents, colorants, and combinations comprising one or more of the foregoing additives.

The formulations can be prepared into tablets or capsules as described further herein.

Optionally, the tablet is coated with a film coating that may or may not contain a colorant. Suitable film coatings include cellulosic materials such as naturally occurring cellulose and synthetic cellulose derivatives such as hydroxypropyl methyl cellulose, methyl cellulose, ethyl cellulose, hydroxypropyl cellulose, hydroxybutyl methyl cellulose, cellulose acetate, cellulose propionate, cellulose acetate propionate, cellulose acetate butyrate, cellulose acetate phthalate, carboxymethyl cellulose, cellulose triacetate, cellulose sulphate sodium salt, and the like; acrylic polymers and vinyl polymers, for example poly(methyl methacrylate), poly (ethyl methacrylate), poly (butyl methacrylate), poly (isobutyl methacrylate), poly (hexyl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene)high density, (poly propylene), poly (ethylene glycol poly (ethylene oxide), poly (ethylene terephthalate), poly(vinyl alcohol), poly(vinyl isobutyl ether), poly(viny acetate), poly (vinyl chloride), polyvinyl pyrrolidone, and the like; proteinaceous materials such as gelatin, polypeptides and natural and synthetic shellacs and waxes; and combinations of the forgoing. The film coating material can be an immediate release coating chosen to provide a coated tablet having substantially the same dissolution properties as the corresponding uncoated tablet. An exemplary film coating material includes Opadry II available from Colorcon.

Generally, when a coating is used, the coating may be used in an amount of about 20 percent by weight or less of the weight of the tablet core; specifically about 15 percent or less; and more specifically about 10 weight percent or less. In one embodiment, the amount of coating material should not be so great that the coating material impedes the release profile of the active agent when ingested or dissolution profile. Thus, it may be possible to use greater than 100 percent of the weight of the tablet core, thereby providing a relatively thick coating.

The term "dosage form" denotes a form that contains an amount sufficient to achieve a therapeutic effect with a single administration. When the formulation is a tablet or capsule, the dosage form is usually one such tablet or capsule. The frequency of administration that will provide the most effective results in an efficient manner without overdosing will vary with the characteristics of the particular active agent, including both its pharmacological characteristics and its physical characteristics such as solubility.

The dosage formulation may comprise the active agent and excipients in the form of particles having a particle size distribution that allows for the ease of processing the material into tablets, by direct compression techniques for example, without segregation of the excipients. The desired particle range of active agent and excipients and other components may be obtained by processes known in the art, including granulating, screening, milling, and the like.

The dosage forms may have a hardness of at least about 5.0 kilopond (kp), specifically at least about 8 kp; more specifically about 10 kp; and yet more specifically about 12 kp. Direct compression techniques are preferred for the formation of the tablets.

When tablets are made by direct compression, the addition of lubricants may be helpful to promote powder flow and to prevent capping of the particle (breaking off of a portion of the particle) when the pressure is relieved. Useful lubricants are as described above including magnesium stearate and hydrogenated vegetable oil (specifically hydrogenated and refined triglycerides of stearic and palmitic acids at about 1% to 5% by weight, specifically about 2% by weight).

In one embodiment, a process of preparing a pharmaceutical dosage formulation comprises blending galantamine or a pharmaceutically acceptable salt thereof with an excipient and disintegrant to form a preblend; blending the preblend with a glidant and optional additives to form a blend; and forming the blend into tablets using direct compression. The process may further comprise blending the preblend with an excipient to form an intermediate blend that is then blended with the glidant.

Suitable methods can be used to apply the film coating to the tablets. Processes such as simple or complex coacervation, interfacial polymerization, liquid drying, thermal and ionic gelation, spray drying, spray chilling, fluidized bed coating, pan coating, electrostatic deposition, may be used. A substantially continuous nature of the coating may be achieved, for example, by spray drying from a suspension or dispersion of the tablet in a solution of the coating composition including a polymer in a solvent in a drying gas having a low dew point.

When a solvent is used to apply the coating, the solvent is preferably an organic solvent that constitutes a good solvent for the coating material, but is substantially a non-solvent or poor solvent for the tablet components, including active agent. The solvent may be selected from alcohols such as methanol, ethanol, halogenated hydrocarbons such as dichloromethane (methylene chloride), hydrocarbons such as cyclohexane, and combinations comprising one or more of the foregoing solvents. Dichloromethane (methylene chloride) has been found to be particularly suitable.

In one embodiment the invention provides a dosage form that exhibits a bio-equivalent profile in accordance to US FDA's guidances. In another embodiment, the Cₘₐₓ value and the area under the plasma concentration-time curve (AUC) from time of administration to 24 hours after administration are from 80 % to 125% of the peak drug concentration (Cₘₐₓ) value and AUC from time of administration to 24 hours after administration exhibited by REMINYL under the same conditions. Also provided herein is a dosage form which exhibits a Cₘₐₓ value and AUC from time of administration to 36 hours after administration that are from 80 % to 125 % of the Cₘₐₓ value and AUC from time of administration to 36 hours after administration exhibited by REMINYL under the same conditions. By "C*ₘₐₓ*" is meant the measured concentration of galantamine in the plasma at the point of maximum concentration. The term "T*ₘₐₓ*" refers to the time at which the concentration of galantamine in the plasma is the highest. "AUC" is the area under the curve of a graph of the concentration of galantamine (typically plasma concentration) vs. time, measured from one time to another.

In an exemplary embodiment, a solid dosage formulation comprises galantamine or a pharmaceutically acceptable salt and is free of microcrystalline cellulose, wherein the formulation provides an AUC after administration that is more than 80 percent and less than 120 percent of the AUC provided between 0 and 24 hours after administration by the same strength dosage form of galantamine hydrobromide wherein the same strength dosage form of galantamine hydrobromide comprises colloidal silicon dioxide in a weight ratio to galantamine hydrobromide of about 0.0234:1, crospovidone in a weight ratio to galantamine hydrobromide of about 0.585:1, hydroxypropylmethylcellulose in a weight ratio to galantamine hydrobromide of about 0.488:1, lactose monohydrate in a weight ratio to galantamine hydrobromide of about 7.53:1, magnesium stearate in a weight ratio to galantamine hydrobromide of about 0.0585:1, microcrystalline cellulose in a weight ratio to galantamine hydrobromide of about 2.51:1, propylene glycol in a weight ratio to galantamine hydrobromide of about 0.188:1, talc in a weight ratio to galantamine hydrobromide of about 0.0975:1, and titanium dioxide in a weight ratio to galantamine hydrobromide of about 0.146:1.

Also provided herein is a method of treating a patient with Alzheimer's disease, comprising administering a therapeutically effective amount of a pharmaceutical dosage formulation as described herein to a patient. Specifically, the administration is by oral administration.

### EXAMPLES

The following examples further illustrate the invention but, of course, should not be construed as in any way limiting its scope.

### Example 1. Galantamine hydrobromide tablets: microcrystalline cellulose study and dissolution rates

Three galantamine hydrobromide formulations were prepared to explore the effect of microcrystalline cellulose on tablet dissolution (Table 1.). Galantamine hydrobromide, lactose impalpable, and lactose monohydrate were blended in a mortar for approximately a minute to form a preblend. The remaining components of the formulation, except for magnesium stearate, were combined with the preblend and blended in a V-blender for 10 minutes. Screened magnesium stearate was then added and the final mixture was blended for another 5 minutes.

**Table 1.**

| Components | Amount (milligram) | | |
|---|---|---|---|
| | Formulation A | Formulation B | Formulation C |
| Galantamine hydrobromide | 12.82 | 12.82 | 12.82 |
| Lactose Impalpable | 35.14 | 35.14 | 35.14 |
| Starch 1500 | 74.50 | 30.00 | -- |
| Lactose monohydrate NF (Fast Flo) | 72.04 | 72.04 | 72.04 |
| Microcrystalline cellulose (Avicel PH 101) | -- | 44.50 | 74.50 |
| Crospovidone | 4.00 | 4.00 | 4.00 |
| Magnesium stearate | 1.50 | 1.50 | 1.50 |
| | | | |
| Total weight | 200.0 | 200.0 | 200.0 |

Each formulation from Table 1 was compressed into direct compression tablets and tested for dissolution profiles according to USP dissolution method II (paddle) using 500 milliliter purified water as the dissolution media at 37°C± 0.5°C in Apparatus 2 (USP 23, <711> Dissolution) using a paddle speed of 50 rotations per minute (rpm). High pressure liquid chromatography (HPLC) was used to determine the amount of active agent dissolved. The dissolution results are provided in Table 2 below as concentration of galantamine % (w/w).

**Table 2.**

| Time | Formulation A | Formulation B | Formulation C |
|---|---|---|---|
| (minutes) | (Standard deviation) | (Standard deviation) | (Standard deviation) |
| 0 | 0.0 (0.0) | 0.0 (0.0) | 0.0 (0.0) |
| 5 | 59.9 (13.9) | 75.4 (16.6) | 71.9(2.9) |
| 10 | 98.6 (1.8) | 91.0(6.2) | 80.5 (2.5) |
| 15 | 97.4 (0.7) | 92.9 (3.9) | 85.1 (2.3) |
| 20 | 97.6 (1.6) | 94.2 (2.6) | 87.4 (2.3) |
| 30 | 99.5 (4.5) | 96.4 (1.7) | 90.5 (2.1) |
| 45 | 100.4 (5.7) | 97.6 (1.1) | 93.1 (1.9) |
| 90 | 100.0 (2.4) | 100.0 (0.4) | 100.0 |

As illustrated by the results in Table 2, the formulations without the presence of microcrystalline cellulose provides a rapid and efficient dissolution of galantamine in water. As the amount of microcrystalline cellulose increases from Formulation B to Formulation C, the rate of dissolution decreases significantly. Not wishing to be bound by theory, but it is proposed that the microcrystalline cellulose interacts with the galantamine hydrobromide, thereby hindering the release of the active agent from the tablet under the dissolution conditions.

When the different grades of microcrystalline cellulose were used, for example, the commercially available AVICEL® 101 and AVICEL® 102, the dissolution rates at the studied time points were substantially the same.

### Example 2. Galantamine hydrobromide formulation containing starch

Three galantamine hydrobromide formulations (Table 3) were prepared to explore the effect of Starch 1500, partially pregelatinized maize starch, on tablet compressibility. Galantamine hydrobromide, lactose impalable, and starch are blended in a V-blender for about 10 minutes to form a preblend. The preblend was passed through a mill with a 0040 screen. The screened preblend was then blended with the remaining components, except for the magnesium stearate, in a V blender for 15 minutes. The magnesium stearate was screened and added into the blender for an additional 5 minutes.

**Table 3.**

| Components | Amount (milligram) | | |
|---|---|---|---|
| | D | E | F |
| Galantamine hydrobromide | 64.10 | 64.10 | 64.10 |
| Lactose Impalpable | 150.00 | 75.00 | 150.00 |
| Starch 1500 | 370.50 | 370.50 | 150.00 |
| Lactose monohydrate NF (Fast Flo) | 360.20 | 435.50 | 581.00 |
| Crospovidone | 50.00 | 50.00 | 50.00 |
| Magnesium stearate | 5.00 | 5.00 | 5.00 |
| | | | |
| Total weight | 999.8 | 1000.1 | 1000.1 |

Each formulation was compressed into direct compression tablets using a Kilian LX Tablet press with a 11/32 inch round tool with a standard curvature. Tablets were prepared for each of the following compression forces: 8,12,15, 20, and 23 kiloNewtons (kN) and the compression profile (Table 4) for the formulation F was recorded.

**Table 4.**

| Compression Force (kN) | Hardness (kp) |
|---|---|
| 8 | 2.88 |
| 12 | 6.07 |
| 15 | 9.47 |
| 20 | 10.54 |
| 23 | 13.92 |

Example 3. Galantamine hydrobromide formulation containing crospovidone

Three galantamine hydrobromide formulations (Table 5) were prepared to explore the effect of excipients on tablet compressibility. Galantamine hydrobromide, lactose impalpable, povidone and starch are blended in a V blender for about 10 minutes to form a preblend. The preblend was passed through a fitz mill with a screen. The screened preblend was then blended with the remaining components, except for the magnesium stearate, in a 1.8 cubic foot V blender for 15 minutes. The glidant (magnesium stearate or stearic acid and Cab-O-Sil) was screened and added into the blender for an additional 5 minutes.

Of the excipients explored in the table, Starch 1500 is partially pregelatinized maize starch and LUDIPRESS is a combination of lactose monohydrate and polyvinylpyrrolidone.

**Table 5.**

| Components | Amount (milligram) | | |
|---|---|---|---|
| | G | H | I |
| Galantamine hydrobromide | 64.10 | 64.10 | 64.10 |
| Lactose Impalpable | 142.40 | 150.00 | 150.00 |
| Starch 1500 | 150.00 | 150.00 | -- |
| Lactose monohydrate NF (Fast Flo) | 581.00 | -- | 681.00 |
| Crospovidone | 50.00 | 50.00 | 50.00 |
| Ludipress | -- | 581.00 | 581.00 |
| Povidone K29/32 | -- | -- | 50.00 |
| Cab-O-Sil | 2.50 | -- | -- |
| Stearic acid | 10.00 | -- | -- |
| Magnesium stearate | -- | 5.00 | 5.00 |
| | | | |
| Total weight | 1000.0 | 1000.1 | 1000.1 |

Each formulation was compressed into direct compression tablets using a Kilian Tablet press with an 11/32 inch round tool. Tablets were prepared for each of the following compression forces: 6,10, 15, 20, and 24 kiloNewtons (kN). Each formulation tableted well, The tablet properties of Formulations G-I are provided in Table 6 below with the standard deviation in parenthesis.

**Table 6.**

| n force | Hardness(kp) | N force | Hardness(kp) | | |
|---|---|---|---|---|---|
| (KN) | F (S.D.) | (KN) | G (S.D.) | H (S.D.) | I (S.D.) |
| 8 | 2.88 (0.29) | 6 | 3.59 (0.11) | 1.93 (0.12) | 4.60 (0.29) |
| 12 | 6.07 (0.17) | 10 | 6.58 (0.25) | 5.33 (0.34) | 7.42 (0.43) |
| 15 | 9.47 (0.45) | 15 | 10.66 (0.40) | 8.31 (0.43) | 12.21 (0.46) |
| 20 | 10.54 (0.42) | 20 | 14.65 (0.42) | 9.83 (0.50) | 15.25 (0.59) |
| 23 | 19.92 (0.30) | 24 | 15.93 (0.46) | 9.88 (0.42) | 15.51 (0.65) |

Formulations F, G and I were subjected to a dissolution test according to the procedure in Example 1 (n=3). As can be seen by the results in Table 7, each formulation exhibited a good dissolution profile although Formulation G showed superior dissolution results.

**Table 7.**

| Time (min) | F(S.D.) | | G (S.D.) | | I (S.D.) | |
|---|---|---|---|---|---|---|
| 0 | 0.0 | (0.0) | 0.0 | (0.0) | 0.0 | (0.0) |
| 10 | 94.6 | (2.5) | 98.5 | (1.1) | 87.6 | (14.1) |
| 20 | 96.3 | (2.7) | 97.8 | (1.0) | 96.9 | (2.8) |
| 30 | 98.5 | (2.4) | 99.5 | (1.5) | 99.5 | (0.6) |
| 45 | 97.1 | (2.8) | 99.4 | (1.4) | 99.6 | (0.4) |
| 90 | 100.0 | (2.2) | 100.0 | (1.6) | 100.0 | (0.4) |

### Example 4. Galantamine hydrobromide tablets, effect of disintegrant on tablet formation

Three formulations were prepared to explore the effect of different disintegrants on the tablet formation for starch and microcrystalline cellulose-free galantamine hydrobromide formulation. The formulations were prepared and tableted according to the procedure of Example 1. The three formulations are provided below in Table 8.

**Table 8.**

| Ingredients | J | K | L |
|---|---|---|---|
| Galantamine HBr | 6.41 | 6.41 | 6.41 |
| Lactose Impalpable | 30.00 | 30.00 | 30.00 |
| Lactose Monohydrate NF (Fast Flo) | 58.09 | 58.09 | 58.09 |
| Crospovidone | 5.00 | - | - |
| Croscarmellose sodium (Ac-Di-Sol) | - | - | 5.00 |
| Sodium starch glycolate (Explo Tab) | - | 5.00 | - |
| Mg Stearate, NF | 0.50 | 0.50 | 0.50 |
| | 100.0 | 100.0 | 100.00 |

The tableted formulations were compressed at forces listed in Table 9. Only Formulation K showed minor lamination at high tableting pressure.

**Table 9.**

| Compression | Hardness (kp) | | | | | |
|---|---|---|---|---|---|---|
| Force (KN) | J (S.D.) | | K (S.D.) | | L (S.D.) | |
| 6 | 4.23 | (0.34) | 4.37 | (0.29) | 4.23 | (0.24) |
| 10 | 8.25 | (0.26) | 7.73 | (0.21) | 8.38 | (0.49) |
| 15 | 12.18 | (0.19) | 11.66 | (0.49) | 11.51 | (0.39) |
| 20 | 15.49 | (0.55) | 12.18* | (0.71) | 12.30 | (0.84) |

| | | | | | | |
|---|---|---|---|---|---|---|
| * Laminate were observed on these samples during hardness testing | | | | | | |

### Example 5. Galantamine hydrobromide tablet properties

Three strengths of a galantamine formulation were prepared into tablets, the formulation is provided in Table 10 with amounts in mg.

**Table 10.**

| | Formulation M | | |
|---|---|---|---|
| Ingredients | (4 mg) | (8 mg) | (12 mg) |
| Galantamine HBr | 5.1 | 10.3 | 15.4 |
| Lactose Impalpable | 24.0 | 48.0 | 72.0 |
| Lactose Monohydrate NF (Fast Flo) | 46.5 | 92.9 | 139.4 |
| Crospovidone | 4.0 | 8.0 | 12.0 |
| Mg Stearate, NF | 0.4 | 0.8 | 1.2 |
| | 80 | 160 | 240 |

Friability: Twenty tablets were accurately weighed and placed in a commercially available friabilator (Distek Friabilator DF-3) and subjected to 200 rotations at 25 rpm speed. The tablets were weighed again after the testing and the percent loss in weight was recorded as friability. The friability was measured on the minimum hardness samples of each batch.

The disintegration time of the tablets was also tested. The disintegration, time was measured according to USP method <701>. Water was the medium. Six tablets were tested for each sample. Record time for last tablet completely disintegrated. The results of the friability test and the disintegration time of each formulation is provided in Table 11 below.

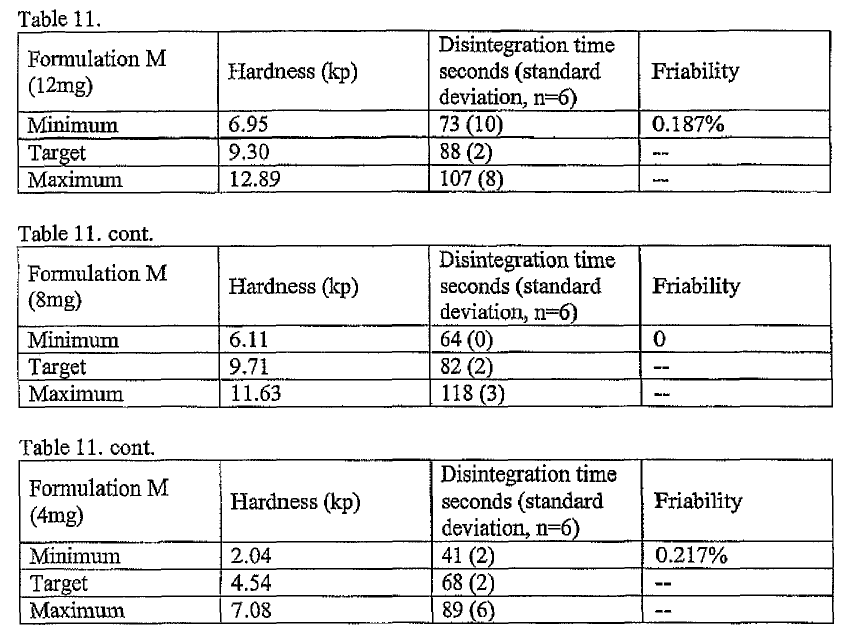

The tablets formed showed very little friability.

### Example 6: Dissolution for coated tablets of Formulation M (12 mg)

Galantamine tablets were examined for dissolution profiles for coated and uncoated tablets of Formulation M (12 mg) described above in Example 5. Tablets were coated using an Aerometic fluid bed. The coating was Opadry II White, an aqueous coating system containing hypromellose, titanium dioxide, triacetin, polydextrose, polyethylene glycol, yellow iron oxide and red iron oxide. Dissolution data on coated and uncoated tablets is provided in Table 12 for 4mg strength tablets (normalized data).

**Table 12.**

| Time | Form. Cores | Form. Coated |
|---|---|---|
| (minutes) | (Standard deviation) | (Standard deviation) |
| 0 | 0.0 (0.0) | 0.0 (0.0) |
| 5 | 84.1 (15.1) | 86.6 (10.8) |
| 10 | 99.0 (3.8) | 98.6 (2.1) |
| 15 | 100.8 (0.3) | 99.3 (1.1) |
| 20 | 100.7 (0.3) | 99.4 (0.8) |
| 30 | 100.5 (0.3) | 99.3 (1.1) |
| 45 | 100.2 (0.3) | 98.7 (0.6) |
| 90 | 100.0 (0.3) | 100.0 (0.8) |

As the results indicate in Table 11, the use of a water soluble coating, such as Opadry II, had little effect on the dissolution properties of the coated tablet as compared to the uncoated tablet.

### Example 7. Galantamine hydrobromide tablets free of microcrystalline cellulose and Starch 1500

Tablets were prepared from the following formulation in Table 13 and tested for tablet properties and dissolution profiles.

**Table 13.**

| Formulation N Components | Amount per gram (mg) |
|---|---|
| Galantamine hydrobromide | 64.08 |
| Lactose monohydrate NF (Impalpable) | 300 |
| Lactose monohydrate NF (Fast Flo) | 116.92 |
| Crospovidone NF (Polyplasdone XL) | 50.00 |
| Lactose monohydrate NF (Fast Flo) | 464 |
| Magnesium stearate | 5.00 |
| | |
| Total weight | 1000 |

The tablets were prepared by charging a V-blender with the following items: lactose monohydrate impalpable, galantamine hydrobromide, lactose monohydrate Fast Flo, crospovidone, and blending for 20 minutes to form a prebled. The preblend was combined with the remaining lactose monohydrate Fast Flo through an auger-fed conventional Hammer Impact mill. The preblend was charged to a V-blender and blended for 20 minutes. Magnesium stearate was screened and added to the V-blender and blended for 5 minutes. The resulting formulations was compressed into three strengths on a tablet press with the following parameter and limits as set forth in Table 14.

**Table 14.**

| | | | |
|---|---|---|---|
| Strength | Form. N, 4mg | Form. N, 8mg | Form. N, 12mg |
| Batch size | 5.8 kg/72k units | 11.6 kg/72k units | 17.2 kg/72k units |
| Tooling | 0.2383 inch round D/A concave | 0.3000 inch round D/A concave | 11/32 inch round D/A concave |
| Run speed | 100K units/hour | 100K units/hour | 100K units/hour |

The tablets were also measured for hardness, thickness, and weight and are provided in Table 15.

**Table 15.**

| Property | Form. N, 4mg | Form. N, 8mg | Form. N, 12mg |
|---|---|---|---|
| | Average of 30 samples; standard deviation; % C.V. | | |
| Thickness (inch) | 0.1080; 0.0004; 0.34 | 0.1354; 0.0005; 0.36 | 0.1572; 0.0005; 0.30 |
| Weight (milligrams) | 79.8000; 0.6644; 0.83 | 159.6333; 0.8899; 0.56 | 240.1200; 1.2689; 0.53 |
| Hardness (kilopond) | 6.5037; 0.4941; 7.60 | 10.8273; 0.7436; 6.87 | 12.3960; 0.8218; 6.63 |

The tablets were tested for dissolution according to the procedure of Example 1 and the results are provided in Table 16.

**Table 16.**

| Time (minutes) | Form. N, 4 mg | | Form. N, 8mg | | Form. N, 12 mg | |
|---|---|---|---|---|---|---|
| | Average (% w/w) | %RSD | Average (% w/w) | %RSD | Average (%w/w) | %RSD |
| 5 | 85.9 | 9.1 | 82.6 | 10.8 | 80.8 | 10.0 |
| 10 | 99.9 | 1.2 | 101.1 | 1.7 | 96.4 | 4.1 |
| 15 | 101.1 | 1.4 | 102.3 | 0.5 | 102.6 | 0.7 |
| 20 | 101.6 | 1.3 | 102.5 | 0.3 | 103.6 | 0.3 |
| 30 | 101.5 | 1.4 | 102.5 | 0.2 | 103.7 | 0.4 |

As illustrated by the foregoing examples, good tablet formation of galantamine hydrobromide formulations can be provided having both good tablet properties as well as good dissolution rates in the absence of microcrystalline cellulose

### Example 8. Lactose-free Formulations

Several formulations are prepared to provide lactose-free galantamine hydrobromide tablets. Formulations are provided in Table 17 in parts by weight.

**Table 17.**

| Components | Amount (weight) | | |
|---|---|---|---|
| | Formulation O | Formulation P | Formulation Q |
| Galantamine hydrobromide | 6.41 | 6.41 | 6.41 |
| Partially pregelatinized starch | 40.0 | - | 30.0 |
| Starch 1500 | - | 40.0 | - |
| Sucrose | 18.09 | 18.09 | |
| Maltodextrin | - | - | 40.0 |
| Mannitol | - | - | 13.09 |
| Crospovidone | - | - | 5 |
| Croscarrmellose sodium | 5.0 | - | - |
| Sodium starch glycolate | - | 5.0 | - |
| Calcium Carbonate | 30.0 | - | - |
| Dibasic Calcium phosphate | Calcium | 30.0 | - |
| Magnesium stearate | 0.5 | 0.75 | - |
| Talc | - | - | 5 |
| Total weight | 100 | 100.25 | 99.5 |

All references, including publications, patent applications, and patents, cited herein are hereby incorporated by reference to the same extent as if each reference were individually and specifically indicated to be incorporated by reference and were set forth in its entirety herein.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Paragraphs of the Invention:
1. A pharmaceutical solid dosage formulation, comprising:
   galantamine or a pharmaceutically acceptable salt thereof; and
   a pharmaceutically acceptable excipient, with the proviso that the formulation does not contain microcrystalline cellulose; and
   wherein the formulation exhibits a dissolution profile such that after 10 minutes at least about 90% of the galantamine or galantamine salt is released after combining the dosage formulation with 500 ml of purified water at 37°C in Apparatus 2 (USP, < 711 > Dissolution, paddle, 50 rpm).
2. The formulation of paragraph 1, wherein the galantamine salt is galantamine hydrobromide.
3. The formulation of paragraph 1, wherein the excipient is selected from the group consisting of lactose-based material; cellulosic material, excluding microcrystalline cellulose; starch; sugar; sugar alcohol; saccharide; polysaccharide; dibasic calcium phosphate, calcium sulfate, and combinations thereof.
4. The formulation of paragraph 1, further comprising a disintegrant.
5. The formulation of paragraph 4, wherein the disintegrant is selected from the group consisting of partially pregelatinized starch, pregelatinized starch, polyvinylpyrrolidone, croscarmellose, croscarmellose sodium, sodium starch glycolate, crospovidone, and combinations thereof.
6. The formulation of paragraph 1, wherein the formulation provides an AUC after administration that is more than 80 percent and less than 120 percent of the AUC provided between 0 and 24 hours after administration by the same strength dosage form of galantamine hydrobromide
   wherein the same strength dosage form of galantamine hydrobromide comprises colloidal silicon dioxide in a weight ratio to galantamine hydrobromide of about 0.0234:1,
   crospovidone in a weight ratio to galantamine hydrobromide of about 0.585:1,
   hydroxypropyl methylcellulose in a weight ratio to galantamine hydrobromide of about 0.488:1,
   lactose monohydrate in a weight ratio to galantamine hydrobromide of about 7.53:1,
   magnesium stearate in a weight ratio to galantamine hydrobromide of about 0.0585:1,
   microcrystalline cellulose in a weight ratio to galantamine hydrobromide of about 2.51:1,
   propylene glycol in a weight ratio to galantamine hydrobromide of about 0.188:1,
   talc in a weight ratio to galantamine hydrobromide of about 0.0975:1, and
   titanium dioxide in a weight ratio to galantamine hydrobromide of about 0.146:1.
7. A tablet comprising the formulation of paragraph 1.
8. The tablet of paragraph 7, further comprising a film coating disposed on the surface of the tablet.
9. A pharmaceutical solid dosage formulation, comprising:
   galantamine or a pharmaceutically acceptable salt thereof; and
   a pharmaceutically acceptable excipient,
   with the proviso that the solid dosage formulation, excluding an optional coating disposed on the solid dosage formulation,
      i) does not contain a cellulosic material, or
      ii) does not contain starch.
10. The formulation of paragraph 9, wherein the galantamine salt is galantamine hydrobromide.
11. The formulation of paragraph 9, wherein the excipient is selected from the group consisting of lactose-based material; sugar; sugar alcohol; saccharide; polysaccharide; dibasic calcium phosphate, calcium sulfate, and combinations thereof.
12. The formulation of paragraph 9, further comprising a disintegrant.
13. The formulation of paragraph 12, wherein the disintegrant is selected from the group consisting of polyvinylpyrrolidone, croscarmellose, croscarmellose sodium, crospovidone, and combinations thereof.
14. A tablet comprising the formulation of paragraph 9.
15. An immediate release solid pharmaceutical dosage formulation, comprising:
   galantamine or a pharmaceutically acceptable salt thereof; and
   a pharmaceutically acceptable excipient, with the proviso that the formulation does not contain a lactose-based material and wherein the formulation is directly compressible.
16. The formulation of paragraph 15, wherein the galantamine salt is galantamine hydrobromide.
17. The formulation of paragraph 16, wherein the excipient is selected from the group consisting of cellulosic material, excluding microcrystalline cellulose; starch; sugar; sugar alcohol; saccharide; polysaccharide; dibasic calcium phosphate, calcium sulfate, and combinations thereof.
18. The formulation of paragraph 15, further comprising a disintegrant.
19. A tablet comprising the formulation of paragraph 15.
20. A process of preparing a pharmaceutical solid dosage formulation, comprising:
   blending galantamine or a pharmaceutically acceptable salt thereof with an excipient and disintegrant to form a preblend;
   blending the preblend with a glidant and optional additives to form a blend; and
   forming the blend into tablets using direct compression; wherein the solid dosage formulation is free of an excipient selected from the group consisting of microcrystalline cellulose, lactose, starch, and combinations thereof.
21. The process of paragraph 20, further comprising blending the preblend with a an excipient to form an intermediate blend that is then blended with the glidant.
22. A pharmaceutical solid dosage formulation, comprising:
   (a) galantamine hydrobromide;
   (b) lactose-based excipient; and
   (c) crospovidone, partially pregelatinized maize starch or a combination of the foregoing; and wherein the solid dosage formulation is free of microcrystalline cellulose.
23. The formulation of paragraph 22, wherein the lactose-based excipient comprises lactose impalpable and spray dried lactose monohydrate.
24. A tablet comprising the formulation of paragraph 22.
25. A method of treating a patient with Alzheimer's disease, comprising orally administering a therapeutically effective amount of the formulation of paragraph 1 to a patient.
26. A method of treating a patient with Alzheimer's disease, comprising orally administering a therapeutically effective amount of the formulation of paragraph 9 to a patient.
27. A method of treating a patient with Alzheimer's disease, comprising orally administering a therapeutically effective amount of the formulation of paragraph 15 to a patient.
28. A method of treating a patient with Alzheimer's disease, comprising orally administering a therapeutically effective amount of the formulation of paragraph 22 to a patient.

## Claims

1. A pharmaceutical solid dosage formulation, comprising:
galantamine or a pharmaceutically acceptable salt thereof; and
a pharmaceutically acceptable excipient,
with the proviso that the solid dosage formulation, excluding an optional coating disposed on the solid dosage formulation,
i. does not contain starch, or
ii. does not contain a cellulosic material.

2. The formulation of claim 1, wherein the galantamine salt is galantamine hydrobromide.

3. The formulation of claim 1, wherein the excipient is selected from the group consisting of lactose-based material ; sugar; sugar alcohol; saccharide; polysaccharide; dibasic calcium phosphate, calcium sulfate, and combinations thereof.

4. The formulation of claim 1, further comprising a disintegrant.

5. The formulation of claim 4, wherein the disintegrant is selected from the group consisting of polyvinylpyrrolidone, croscarmellose, croscarmellose sodium, crospovidone, and combinations thereof.

6. A tablet comprising the formulation of claim 1.

7. A tablet according to claim 6, further comprising a film coating disposed on the surface of the tablet.

8. The formulation according to any of claims 1 to 7 for use in the treatment of Alzheimer's disease.
